# EUROPEAN PATENT APPLICATION

(11) **EP 4 574 841 A1**
(43) Date of publication of application: **25.06.2025**
(21) Application number: 23217931.7
(22) Date of filing: 19.12.2023
(51) Int. Cl.: C07K 16/12, C07K 16/28

(54) **ANTI-BACTERIAL POLYPEPTIDES**

(71) Applicant: BioNTech SE, 55131 Mainz (DE)
(72) Inventor: SCHÜBEL, Marisa, 55131 Mainz (DE); JACOMET, Hippolyte, 55131 Mainz (DE); TRAORE, Seydou, 55131 Mainz (DE); SAHIN, Ugur, 55131 Mainz (DE); BECKMANN, Karsten, 55131 Mainz (DE); CARLE, Anna, 55131 Mainz (DE)
(74) Representative: D Young & Co LLP

(57) **Abstract**

The present invention relates to mutated polypeptides comprising a fragment crystallisable (Fc) region with reduced affinity for *Staphylococcus aureus* protein A (SpA). The present invention further relates to the treatment of *Staphylococcus aureus* infections.

## Description

### Field of the invention

The present invention relates to mutated polypeptides comprising a fragment crystallisable (Fc) region with reduced affinity for *Staphylococcus aureus* protein A (SpA). The present invention further relates to the treatment of *Staphylococcus aureus* infections.

### Background of the invention

*Staphylococcus aureus* (*S. aureus*) is a bacterium commonly found on the skin and mucous membranes of humans, which is a significant concern in healthcare settings. While it can exist harmlessly as a part of the normal microbiota, it is also capable of causing a range of infections and health issues. *S*. *aureus* infections can take various forms, including skin and soft tissue infections (such as boils, abscesses, and cellulitis), respiratory infections, bloodstream infections, pneumonia, endocarditis (infection of the heart valves) and sepsis. *S. aureus* produces a wide array of virulence factors, which contribute to its ability to evade the immune system and cause damage to host tissues. Some of these factors can also reduce the effectiveness of therapeutic antibodies (and other associated immunotherapies).

In particular, *S. aureus* expresses Staphylococcal Protein A (SpA), an IgG-binding protein that binds human IgG antibodies via their Fc-domain. *S. aureus* further expresses some additional IgG-binding proteins which also bind human IgG-antibodies via their Fc-domain: staphylococcal binder of immunoglobulins (Sbi). An equivalent protein in *Streptococcus* spp. is Streptococcal Protein G (SpG). Due to the abundant surface expression of SpA (and to a lesser extent Sbi), *S. aureus* essentially coats itself in human IgG-antibodies, thereby not only obscuring its own antigens but also effectively inhibiting Fc-mediated effector functions of the antibodies. This ability of *S. aureus* affects proximal human IgG antibodies and other Fc-containing proteins in general. Thus, chiefly through the expression of SpA, *S. aureus* reduces the effectiveness of therapeutic antibodies and other related Fc-containing immunotherapies. For this reason, for an effective use of a therapeutic antibody (or related immunotherapy) in *S. aureus* infections, the Fc-mediated capture of the therapeutic antibody etc. must be inhibited.

Chen et al., PNAS 119(4) e2114478119 (2022) reported some preliminary alleged results into the possibility of mutating an antibody Fc region to reduce SpA affinity, but the investigators encountered difficulties in maintaining the properties of the Fc regions with the mutations that were introduced. Two mutated Fc regions for this purpose are disclosed in Chen et al.: "AESP" with the mutations S254A, Q311E, L432S and N434P, and "R" with the mutation H435R. However, the corresponding patent publication US 2023/0041644 A1 by the same group indicates that AESP had unfavourable properties, which precluded further development. Chen *et al.* further discloses the mutations T307R and A378V in combination with the H435R mutation. US 2020/0291099 A1 discloses several Fc mutations for allegedly reducing SpA affinity, but only discloses that a single antibody "Mab2", with the mutations K274Q, H435R and Y436F was considered for being carried through to preliminary testing.

Thus, there remains a need in the art for Fc mutations and combinations thereof to provide Fc region polypeptides with reduced affinity to SpA, for use against *Staphylococcus aureus* infections. The identification of such Fc mutations and Fc region polypeptides is far from straightforward, as there is a need to balance many different factors such as production capability, stability and reduced SpA affinity. There is also a need in the art for the Fc mutants to have advantageous FcRn binding characteristics for antibody half-life (i.e. binding at pH 6 and weak binding at pH 7), as well as maintained Fc effector functions through FcγR interactions.

### Summary of the invention

The present invention relates to mutations in the Fc region of polypeptides for therapeutic or diagnostic use in *Staphylococcus aureus* infections. In particular, the present invention provides polypeptides comprising novel substitution mutations and combinations thereof in the Fc region, which are selected from T307I, Q311R, M428L, N434L, Y436K and combinations thereof. The polypeptides of the invention have reduced affinity for *Staphylococcus aureus* protein A (SpA), as well as other advantageous properties such as maintained stability and expressibility. Polypeptides of the invention also have favourable properties regarding Fc function, such as a maintained immune response, retained C1q binding and even improved complement component 1q (C1q) recruitment. Moreover, polypeptides of the present invention have favourable properties regarding FcRn binding and antibody half-life. Furthermore, the polypeptides of the present invention are useful in treating and/or diagnosing *Staphylococcus aureus* infections.

Accordingly, in an aspect, the present invention provides a polypeptide comprising a fragment crystallisable (Fc) region having reduced affinity for *Staphylococcus aureus* protein A (SpA), wherein the polypeptide sequence of the Fc region comprises one or more of the following substitution mutations: T307I, Q311R, M428L, N434L and Y436K.

In an aspect, the present invention provides one or more nucleic acid sequences capable of expressing the polypeptide according to the invention.

In an aspect, the present invention provides a cell comprising the polypeptide or the one or more nucleic acid sequences according to the invention.

In an aspect, the present invention provides a composition comprising the polypeptide, the one or more nucleic acid sequences or the cell according to the invention.

In an aspect, the present invention provides an *in vitro* method comprising contacting a cell with the polypeptide, the one or more nucleic acid sequences, the cell or the composition of the invention.

In an aspect, the present invention provides the polypeptide, the one or more nucleic acid sequences, the cell or the composition of the invention for use in a method of therapy or a diagnostic method.

In an aspect, the present invention provides the polypeptide, the one or more nucleic acid sequences, the cell or the composition of the invention for use in the manufacture of a medicament for a therapy or a diagnostic agent.

In an aspect, the present invention provides a method of therapy or a diagnostic method comprising administering the polypeptide, the one or more nucleic acid sequences, the cell or the composition of the invention to a subject.

In an aspect, the present invention provides a method for making the polypeptide according to the invention, comprising expressing one or more nucleic acids capable of expressing the polypeptide.

### Brief description of the Figures

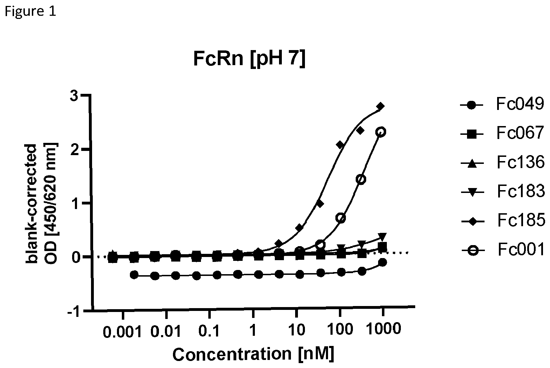

**Figure 1****:** FcRn binding of Fc mutants at pH 7, tested via ELISA. For efficient antibody recycling within the cell, it is advantageous for FcRn binding to be weak at pH 7. Fc185 shows strong binding to FcRn at pH 7. Binding to FcRn at pH7 is acceptable for all other tested mutants.

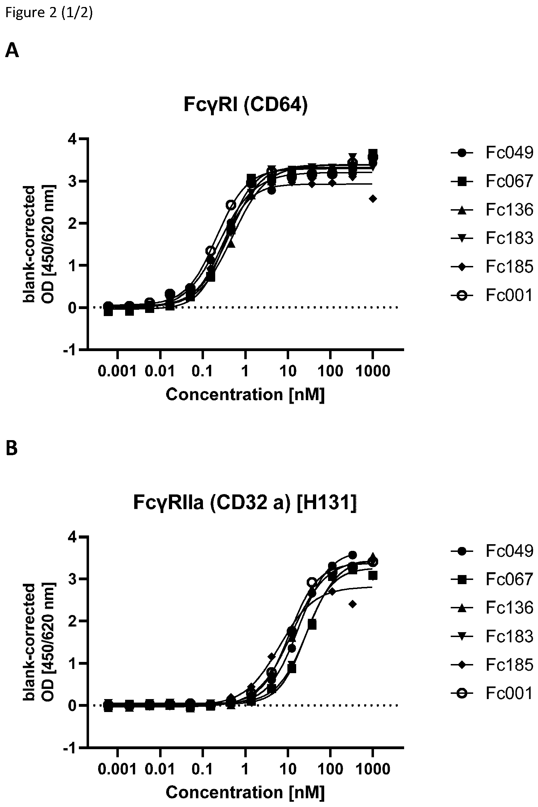

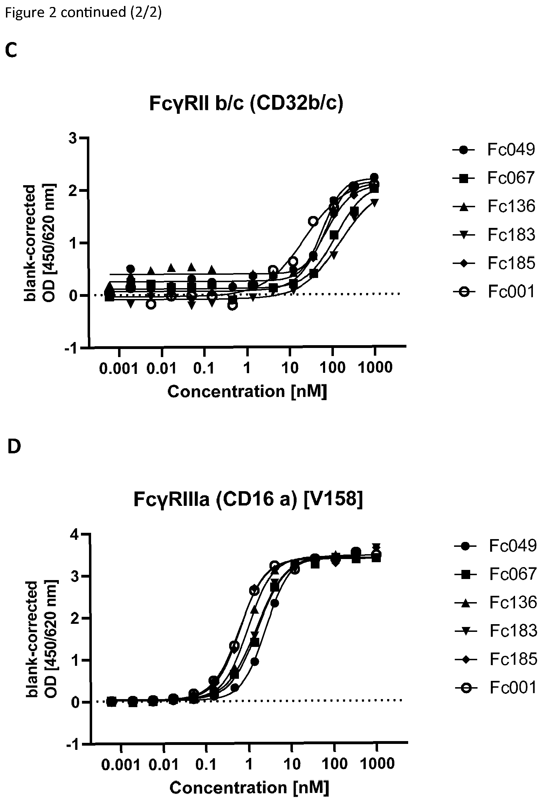

**Figure 2****:** FcγR binding of Fc mutants, tested via ELISA in respect of various different receptors, including FcγRl (Figure 2A), FcγRlla (Figure 2B), FcγRllb/c (Figure 2C) and FcγRllla (Figure 2D) Binding to Fcγ-Receptors for the screening candidate mutant Fc136 was comparable to the wildtype control.

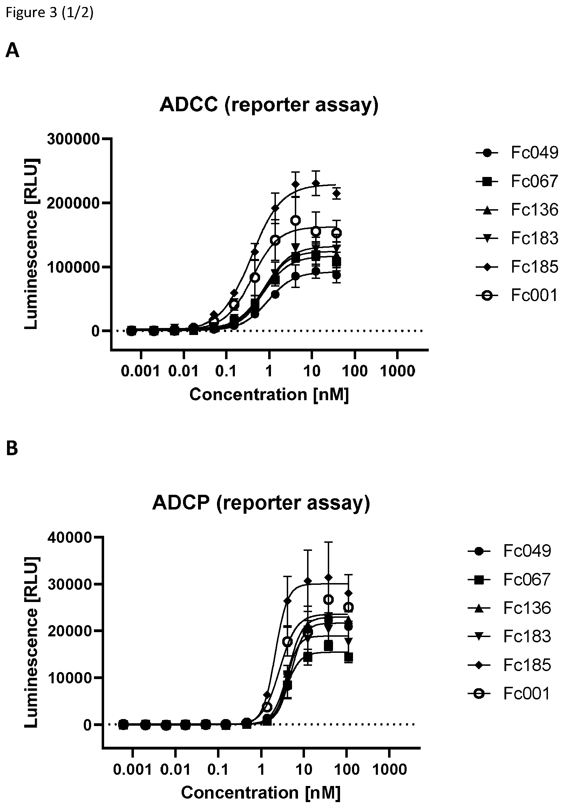

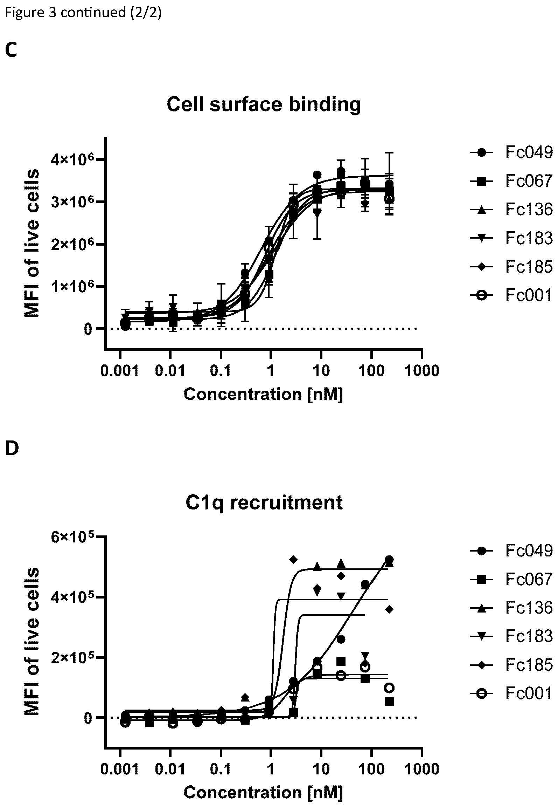

**Figure 3****:** Functional testing of Fc mutants, including assays for ADCC (Figure 3A), ADCP (Figure 3B), cell surface binding (Figure 3C) and C1q recruitment (Figure 3D). Fc136 performed comparably to the wildtype protein in Figures 3A-3C, and surprisingly showed favourably strong signals in the C1q recruitment assay in Figure 3D.

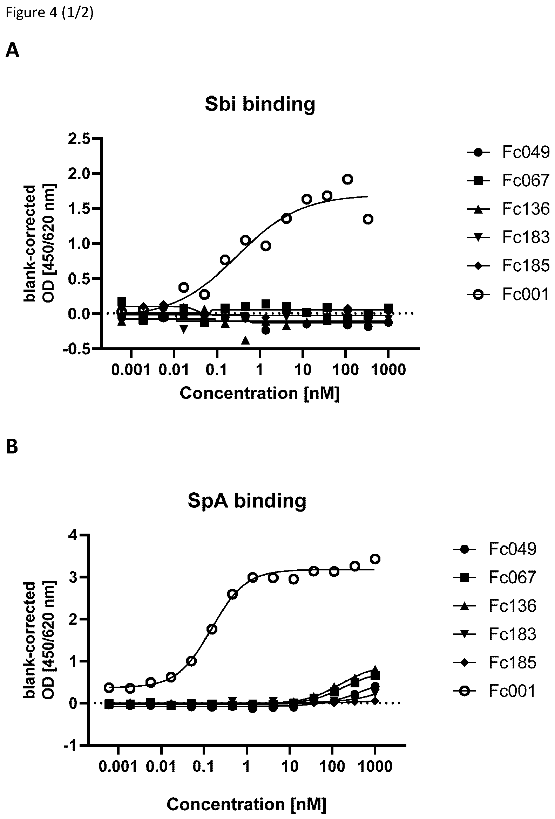

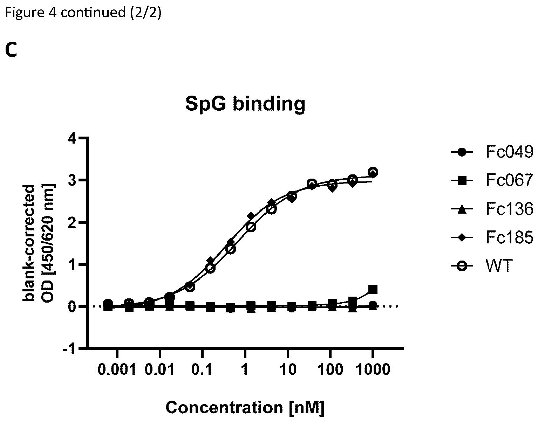

**Figure 4****:** Binding assay testing of Fc mutants, including Sbi binding assays (Figure 4A), SpA binding assays (Figure 4B) and SpG binding assays (Figure 4C) for several Fc mutants as well as a wild-type control (Fc001/WT). Sbi, SpA and SpG did not substantially bind to Fc136. To the contrary, SpG did show binding to the Fc185 mutant.

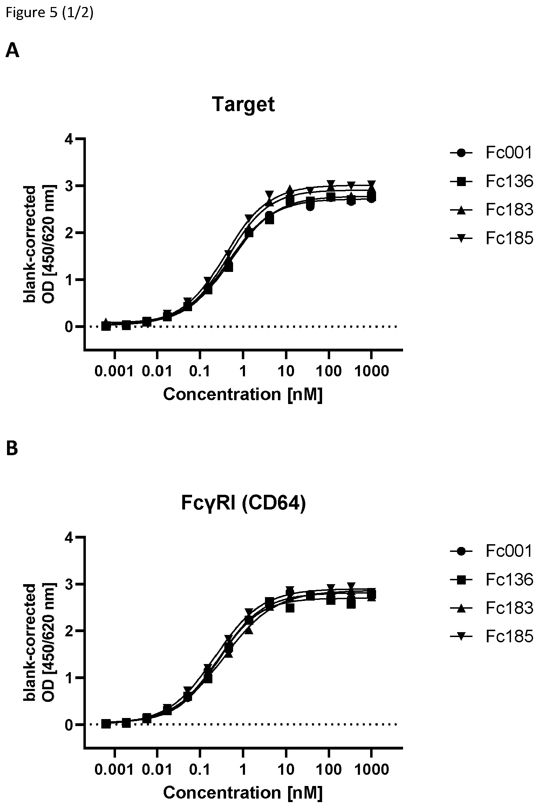

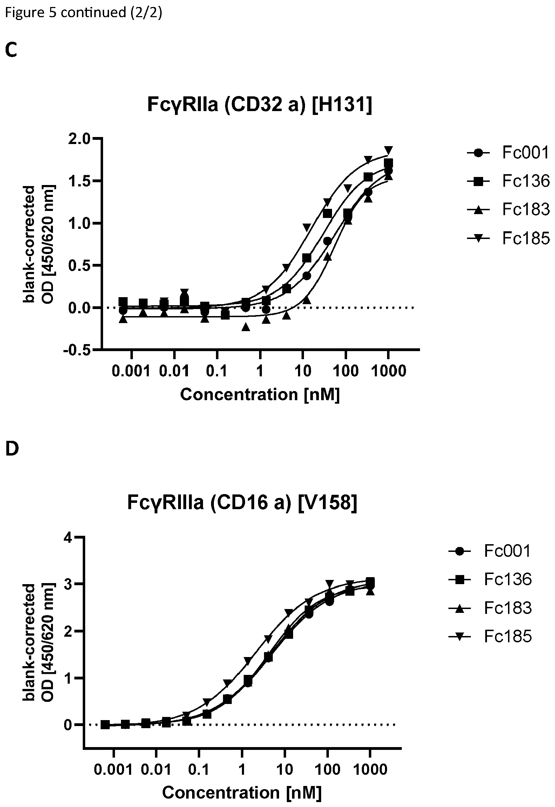

**Figure 5****:** Binding assays for the mutants Fc136, Fc183 and Fc185, as well as a wild-type control (Fc001) in highly purified forms, to the target antigen (Figure 5A), FcγRlla (Figure 5B), FcγRl (Figure 5C) and FcγRllla (Figure 5D).

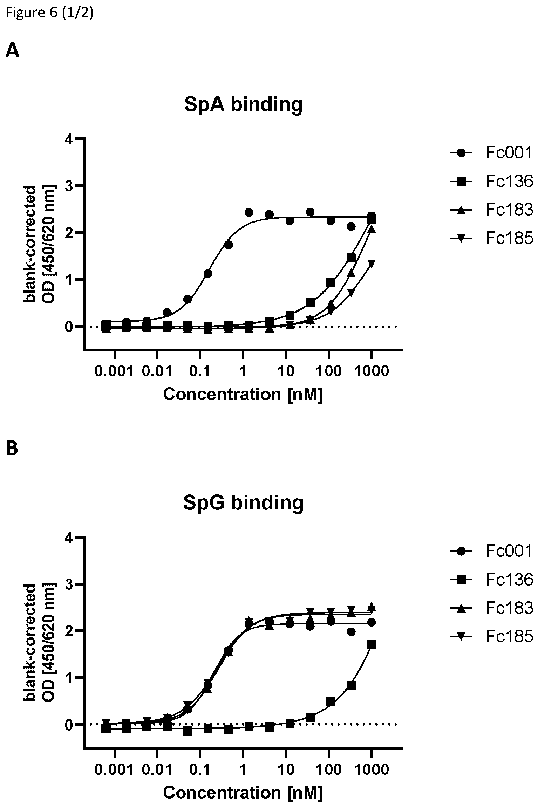

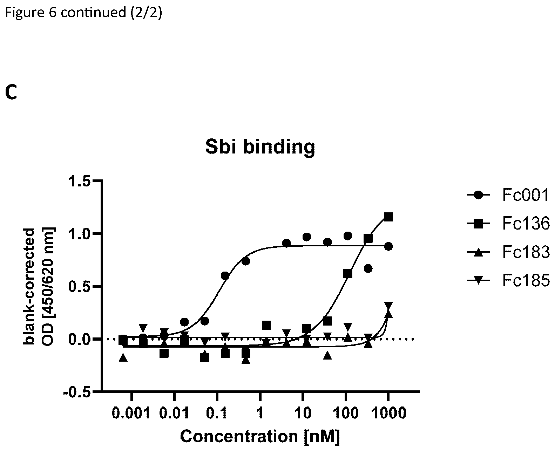

**Figure 6****:** Binding assays for the mutants Fc136, Fc183 and Fc185, as well as a wild-type control (Fc001/WT) in highly purified forms, to SpA (Figure 6A), SpG (Figure 6B) and Sbi (Figure 6C). SpA, SpG and Sbi did not substantially bind to Fc136.

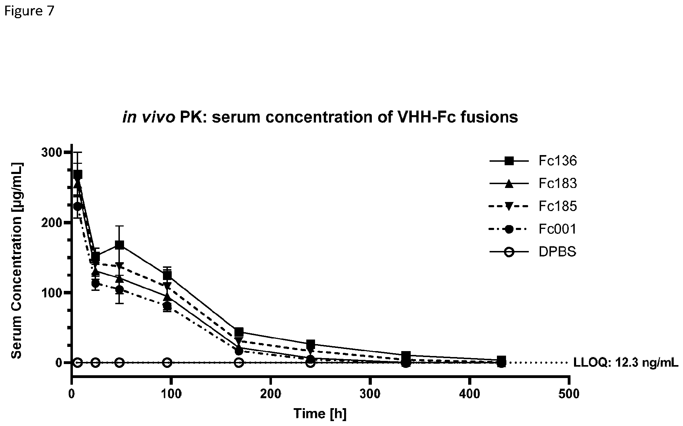

**Figure 7****:** Serum concentrations of VHH-Fc-fusions in NSG hFcRn (32) Tg mice over time. Serum concentrations of the mutants Fc136, Fc183, Fc185, as well as the wild-type control (Fc001) and a negative control (DPBS) over time. Fc136 shows consistently higher serum concentration than all other samples, indicative of a prolonged *in vivo* half-life.

### Detailed description of the invention

### Polypeptides and mutations

In an aspect, the present invention provides a polypeptide comprising a fragment crystallisable (Fc) region having reduced affinity for *Staphylococcus aureus* protein A (SpA), wherein the polypeptide sequence of the Fc region comprises one or more of the following substitution mutations: T307I, Q311R, M428L, N434L and Y436K.

In an embodiment, the polypeptide sequence of the Fc region comprises one substitution mutation. In an embodiment, the substitution mutation is T307I, Q311R, M428L, N434L or Y436K.

In an embodiment, the polypeptide sequence of the Fc region comprises two substitution mutations. In an embodiment, the polypeptide sequence of the Fc region comprises the substitution mutations T307I and Q311R; T307I and M428L; T307I and N434L; T307I and Y436K; Q311R and M428L; Q311R and N434L; Q311R and Y436K; M428L and N434L; M428L and Y436K, or N434L and Y436K.

In an embodiment, the polypeptide sequence of the Fc region comprises three substitution mutations. In an embodiment, the polypeptide sequence of the Fc region comprises the substitution mutations T307I, Q311R, and M428L; T307I, Q311R, and N434L; T307I, Q311R, and Y436K; T307I, M428L, and N434L; T307I, M428L, and Y436K; T307I, N434L, and Y436K; Q311R, M428L, and N434L; Q311R, M428L, and Y436K; orQ311R, N434L, and Y436K.

In an embodiment, the polypeptide sequence of the Fc region comprises four substitution mutations. In an embodiment, the polypeptide sequence of the Fc region comprises the substitution mutations T307I, Q311R, M428L, and N434L; T307I, Q311R, M428L, and Y436K; T307I, Q311R, N434L, and Y436K; T307I, M428L, N434L, and Y436K; or Q311R, M428L, N434L, and Y436K.

In an embodiment, the polypeptide sequence of the Fc region comprises the substitution mutation M428L, and optionally comprises one or more of T307I, Q311R, N434L and Y436K. As a mutation that is not only novel but at an entirely novel position, and which was also identified in just a single variant in the screening carried out by the present inventors, the substitution mutation M428L is of particular interest. Thus, in a generally preferred embodiment herein, the polypeptide sequences of the Fc region comprises (at least) the substitution mutation M428L.

In an embodiment, the polypeptide sequence of the Fc region comprises five substitution mutations. In a most preferred embodiment, the polypeptide sequence of the Fc region comprises all of the substitution mutations T307I, Q311R, M428L, N434L and Y436K.

In an embodiment, the polypeptide sequence of the Fc region comprises a sequence having at least 70%, 75%, 80%, 85%, 90%, 95%, 96%, 97%, 98%, 99% or 100% identity to SEQ ID NO: 3. In an embodiment, the % identity language does not apply to the substitution mutations that are specified in the Fc region of the polypeptide. In an embodiment, the polypeptide sequence of the Fc region comprises SEQ ID NO: 3.

In an embodiment, the polypeptide of the Fc region does not comprise the substitution mutation H435R. In an embodiment, the polypeptide of the Fc region does not comprise the substitution mutations S254A, Q311E, L432S or N434P. In an embodiment, the polypeptide of the Fc region does not comprise the substitution mutations T307R or A378V. In an embodiment, the polypeptide of the Fc region does not comprise the substitution mutations T307Q, Q311V or A378V. In an embodiment, the polypeptide of the Fc region does not comprise the substitution mutations T256D, N286D, T307R, Q311V or A378V.

It will be understood that the antibody and Fc amino acid numbering herein corresponds to the EU numbering scheme. Thus, in an embodiment, the amino acid position numbering corresponds to EU numbering. The EU numbering scheme, including how it compares to other antibody residue numbering schemes, is well-known in the art and can be viewed at e.g. https://www.imqt.orq/IMGTScientificChart/Numbering/Hu IGHGnber.html.

In some embodiments herein, the mutations are defined simply by the position of the mutation in the Fc region (according to the EU numbering scheme) and the residue which is present. For example, in some embodiments herein the polypeptide having the substitution mutation M428L is simply defined as the polypeptide having the residue "L" at position 428.

In embodiments herein, the mutations T307I, Q311R, M428L, N434L and Y436K may alternatively be defined as the polypeptide sequence of the Fc region comprising "I" at EU position 307, "R" at EU position 311, "L" at EU position 428, "L" at EU position 434, and/or "K" at EU position 436 respectively.

In embodiments herein, the mutations S254A, T256D, N286D, T307R, T307Q, Q311E, Q311V, A378V, L432S, N434P and H435R may alternatively be defined as the polypeptide sequence of the Fc region which does not comprise "A" at EU position 254, "D" at EU position 256, "D" at EU position 286, "R" at EU position 307, "Q" at EU position 307, "E" at EU position 311, "V" at EU position 311, "V" at EU position 378, "S" at EU position 432, "P" at EU position 434 and/or "R" at EU position 435 respectively.

### Polypeptide functions

In an embodiment, the Fc region of the polypeptide has reduced affinity for SpA. In an embodiment, the Fc region of the polypeptide has reduced affinity for the second immunoglobulin-binding protein of *Staphylococcus aureus* (Sbi). In an embodiment, the Fc region of the polypeptide has reduced affinity for Streptococcal protein G (SpG).

Herein, "reduced affinity" of a mutant Fc region for SpA, Sbi and/or SpG means that the affinity is reduced relative to the equivalent wild-type Fc region, i.e. the same Fc region polypeptide sequence which does not comprise any of the mutations that are specified in the claim. It will be understood that SpA, Sbi and SpG typically have affinity for and are able to capture the Fc regions of polypeptides which contain such regions (e.g. antibodies). Thus, an Fc region of the invention with substitution mutations having reduced affinity for SpA, Sbi and/or SpG means that the Fc region has reduced affinity relative to the same Fc region without the substitution mutations. In this context, for example, an Fc region "without the substitution mutation" M428L does not have the residue "L" at EU position 428, and instead has the residue "M" at EU position 428.

Thus, in an embodiment, the Fc region of the polypeptide of the invention has reduced affinity for SpA, Sbi and/or SpG relative to the Fc region of the polypeptide that does not comprise the substitution mutations.

In an embodiment, "reduced" means that SpA, Sbi and/or SpG substantially do not bind to the Fc region of the polypeptide of the present invention. In an embodiment, "reduced" means that SpA, Sbi and/or SpG do not bind to the Fc region of the polypeptide of the present invention. In an embodiment, the Fc region of the polypeptide of the present invention having "reduced affinity" means that the binding of SpA, Sbi and/or SpG to the Fc region is inhibited. In an embodiment, SpA-mediated antibody capture is inhibited. In an embodiment, the reduced affinity for SpA (and/or Sbi) means that Fc effector functions are maintained in the presence of *S*. *aureus* or SpA.

It will be understood that the affinity for SpA, Sbi and/or SpG herein refers to the ability of these proteins to bind to and capture antibodies and other immunoglobulin domain-containing polypeptides by binding to the Fc region (and possibly the VH3 Fab region). This affinity does not include the specific antibody binding of the polypeptides of the present invention, e.g. to SpA, where present. Thus, this affinity does not include the polypeptides of the present invention specifically binding to SpA, Sbi and/or SpG via antibody-like binding, i.e. via the CDRs of one or more variable immunoglobulin domain.

It will also be understood that reduced affinity for SpA, Sbi and/or SpG can be determined by *in silico* modelling/screening and (if necessary) can be further verified by cellular assays.

In an embodiment, the Fc-mediated effector functions of the polypeptide are not inhibited in the presence of *Staphylococcus aureus.*

In an embodiment, the Fc region of the polypeptide of the invention binds to the neonatal Fc receptor (FcRn). In an embodiment, the Fc region of the polypeptide binds to FcRn with greater affinity at pH 6 than at pH 7. In an embodiment, the binding of the Fc region of the polypeptide of the invention to FcRn is equivalent, comparable or similar to that of the Fc region of the polypeptide that does not comprise the substitution mutations of the invention. In an embodiment, the binding affinity of the Fc region of the polypeptide of the invention to FcRn is equivalent, comparable or similar to that of the Fc region of the polypeptide that does not comprise the substitution mutations of the invention. In an embodiment, the binding affinity of the Fc region of the polypeptide of the invention to FcRn at pH 6 and at pH 7 is equivalent, comparable or similar to that of the Fc region of the polypeptide that does not comprise the substitution mutations of the invention. It will be understood that binding to the FcRn receptor can be readily determined and measured, e.g. by ELISA.

In an embodiment, the polypeptide of the invention has an *in vivo* half-life that is at least equivalent to the *in vivo* half-life of the polypeptide comprising an Fc region that does not comprise the substitution mutations of the invention. In an embodiment, the polypeptide has an *in vivo* half-life that is similar to or greater than the *in vivo* half-life of the polypeptide comprising an Fc region that does not comprise the substitution mutations of the invention. In an embodiment, the polypeptide has an *in vivo* half-life that is comparable to or greater than the *in vivo* half-life of the polypeptide comprising an Fc region that does not comprise the substitution mutations of the invention. In an embodiment, the *in vivo* half-life is in a mouse. In an embodiment, the *in vivo* half-life is in a human.

In an embodiment, the Fc region of the polypeptide of the invention binds to the Fc-gamma receptor (FcγR). In an embodiment, the Fc region of the polypeptide binds to FcγRl. In an embodiment, the Fc region of the polypeptide binds to FcγRlla. In an embodiment, the Fc region of the polypeptide binds to FcγRllb/c. In an embodiment, the Fc region of the polypeptide binds to FcγRllla. In an embodiment, the binding of the Fc region of the polypeptide of the invention to FcγR is equivalent to that of the Fc region that does not comprise the substitution mutations of the invention. In an embodiment, the binding of the Fc region of the polypeptide of the invention to FcγRl, FcγRlla, FcγRllb/c and/or FcγRIIIa is equivalent to that of the Fc region that does not comprise the substitution mutations of the invention. It will be understood that binding to FcγR receptors can be readily determined and measured, e.g. by ELISA.

In an embodiment, the polypeptide of the invention induces antibody-dependent cellular cytotoxicity (ADCC). In an embodiment, the polypeptide of the invention induces antibody-dependent cellular phagocytosis (ADCP). In an embodiment, the polypeptide of the invention induces complement-dependent cytotoxicity (CDC). In an embodiment, the polypeptide of the invention induces ADCC, ADCP and CDC. In an embodiment, the polypeptide of the invention induces ADCC and/or ADCP that is equivalent to that of the polypeptide that does not comprise the substitution mutations of the invention.

In an embodiment, the polypeptide of the invention induces complement component 1q (C1q) recruitment. In an embodiment, the polypeptide of the invention has improved C1q recruitment. In an embodiment, the polypeptide of the invention has improved C1q recruitment relative to the polypeptide that does not comprise the substitution mutations of the invention. C1q recruitment to target cells bound by IgG antibodies/VHH-Fc fusion proteins etc. initiates the classical pathway of complement, thereby promoting CDC. Accordingly, in an embodiment, the polypeptide of the invention induces CDC. In an embodiment, the polypeptide of the invention has enhanced CDC induction. In an embodiment, the polypeptide of the invention induces greater CDC than the polypeptide that does not comprise the substitution mutations of the invention.

### Improved half-life

In preferred embodiments, the polypeptides of the invention have been found to possess an *in vivo* half-life that is unexpectedly greater than, e.g. substantially greater than, the *in vivo* half-life of polypeptides comprising an Fc region that does not comprise the substitution mutations of the invention. This also presents a substantial improvement of the polypeptides of the invention over the polypeptides of the prior art.

In further detail, as demonstrated in the Examples herein, the Fc183 control mutant comprises the H435R mutation, but does not possess a substantially greater *in vivo* half-life compared to the equivalent wild-type polypeptide (Fc001). Further, the Fc185 control mutant comprises the H435R, T307R and A378V mutations, but still possesses a lesser *in vivo* half-life to that of Fc136. To the contrary, the Fc136 mutant, i.e. a polypeptide of the present invention, comprises the mutations T307I, Q311R, M428L, N434L and Y436K, and has been determined to possess an *in vivo* half-life that is substantially greater than all of: the equivalent wild-type polypeptide (Fc001), Fc183 and Fc185.

Thus, without wishing to be bound by theory, the present inventors consider that a further advantage of an improved *in vivo* half-life is provided by the mutations that are present in the polypeptides of the present invention (e.g. Fc136), relative to the equivalent wild-type polypeptide of Fc001. Moreover, this improvement of *in vivo* half-life is unexpectedly significantly greater than Fc183 (which shows no difference vis-à-vis Fc001) and Fc185.

Accordingly, in an embodiment, the polypeptide of the invention has an *in vivo* half-life that is greater than, such as substantially greater than, the *in vivo* half-life of a 'control' or 'reference' polypeptide. Accordingly, in an embodiment, the polypeptide of the invention has an *in vivo* half-life that is greater than, such as substantially greater than, the *in vivo* half-life of the polypeptide comprising an Fc region that does not comprise the substitution mutations of the invention. In an embodiment, the polypeptide of the invention has an *in vivo* half-life that is greater than, such as substantially greater than, the *in vivo* half-life of the polypeptide comprising an Fc region that comprises the H435R mutation. In an embodiment, the polypeptide of the invention has an *in vivo* half-life that is greater than, such as substantially greater than, the *in vivo* half-life of the polypeptide comprising an Fc region that comprises the H435R, T307R and A378V mutations. In embodiments, it will be understood that the appropriate 'control' or 'reference' polypeptide does not comprise (e.g. any of the) mutations of the invention. In embodiments, the appropriate 'control' or 'reference' polypeptide does not comprise any mutations that increase the half-life of the polypeptide relative to the wild-type Fc region, e.g. the equivalent wild-type Fc region to the polypeptide of the invention. In embodiments, the appropriate 'control' or 'reference' polypeptide does not comprise any mutations relative to the wild-type Fc region, e.g. the equivalent wild-type Fc region to the polypeptide of the invention.

In an embodiment, the polypeptide of the present invention has an *in vivo* half-life that is greater than a polypeptide comprising an Fc region having the sequence as set forth in SEQ ID NO: 4. In an embodiment, the polypeptide of the present invention has an *in vivo* half-life that is greater than that of Fc183. In an embodiment, the polypeptide of the present invention has an *in vivo* half-life that is greater than a polypeptide comprising an Fc region having the sequence as set forth in SEQ ID NO: 5. In an embodiment, the polypeptide of the present invention has an *in vivo* half-life that is greater than that of Fc185.

In some embodiments herein, an *in vivo* half-life "greater than" means "substantially greater than". In some embodiments herein, it will be understood that an *in vivo* half-life "greater than" means that the polypeptide of the invention is capable of having a greater or more prolonged effect *in vivo* than the relevant 'control' or 'reference' polypeptide.

In some embodiments herein, a polypeptide of the invention having an *in vivo* half-life "greater than" a polypeptide comprising an Fc region that does not comprise the substitution mutations of the invention, e.g. an equivalent wild-type polypeptide (such as Fc001), means at least 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% or 30% greater than a polypeptide comprising an Fc region that does not comprise the substitution mutations of the invention.

In some embodiments herein, a polypeptide of the invention having an *in vivo* half-life that is "greater than" a polypeptide comprising an Fc region that comprises a H435R mutation but does not comprise the substitution mutations of the invention (such as Fc183) means at least 5%, 6%, 7%, 8%, 9%, 10%, 11%, 12%, 13%, 14%, 15%, 16%, 17%, 18%, 19%, 20%, 21%, 22%, 23%, 24%, 25%, 26% or 27% greater than a polypeptide comprising an Fc region that comprises a H435R mutation but does not comprise the substitution mutations of the invention (e.g. Fc183).

In some embodiments herein, an *in vivo* half-life that is "greater than" a polypeptide comprising an Fc region that comprises H435R, T307R and A378V mutations but does not comprise the substitution mutations of the invention (such as Fc185) means at least 5%, 6%, 7%, 8%, 9%, 10%, 11% or 12% greater than a polypeptide comprising an Fc region that comprises H435R, T307R and A378V mutations but does not comprise the substitution mutations of the invention (e.g. Fc185).

In an embodiment, the polypeptide of the present invention has an *in vivo* half-life of at least 71 hours, at least 72 hours, at least 73 hours, at least 74 hours, at least 75 hours, at least 76 hours, at least 77 hours, at least 78 hours, at least 79 hours, at least 80 hours, at least 81 hours, at least 82 hours, at least 83 hours, at least 84 hours, at least 85 hours, at least 86 hours, at least 87 hours, at least 88 hours, at least 89 hours, at least 90 hours, at least 91 hours or at least 92 hours.

In an embodiment, the polypeptide of the present invention has an *in vivo* half-life that is at least equivalent to, substantially the same as or the same as the *in vivo* half-life of a polypeptide comprising a Fc region that comprises the T307I, Q311R, M428L, N434L and Y436K mutations. In an embodiment, the polypeptide of the present invention has an *in vivo* half-life that is at least equivalent to, substantially the same as or the same as the *in vivo* half-life of a polypeptide comprising a Fc region that comprises "I" at position 307, "R" at position 311, "L" at position 428, "L" at position 434 and "K" at position 436. In an embodiment, the polypeptide of the present invention has an *in vivo* half-life that is at least equivalent to, substantially the same as or the same as the *in vivo* half-life of a polypeptide comprising an Fc region having the sequence set forth in SEQ ID NO: 3.

It is to be understood herein that half-life may be considered as a measure of longevity *in vivo.* Thus, in some embodiments, the reference to "*in vivo* half-life" is considered a reference to "*in vivo* longevity". In an embodiment, a "greater" *in vivo* half-life is a more prolonged *in vivo* half-life. In an embodiment, a "greater" *in vivo* half-life means that the time taken for the *in vivo* amount of the polypeptide to drop to half of a pre-determined initial amount is longer. In an embodiment, the *in vivo* half-life is in a human. In an embodiment, the *in vivo* half-life is in a mouse. In an embodiment, the *in vivo* half-life is the serum half-life. In an embodiment, the *in vivo* half-life is the serum half-life in a human. In an embodiment, the *in vivo* half-life is the serum half-life in a mouse. In an embodiment, the *in vivo* half-life is the serum half-life in a mouse model expressing human FcRn instead of murine FcRn.

In some embodiments wherein a comparison is made between the *in vivo* half-life of a polypeptide of the present invention and another polypeptide, it is to be understood that - unless otherwise stated, e.g. except for any stated differences in mutations - the 'reference' polypeptide is the same as, substantially the same as or equivalent to the polypeptide of the present invention.

Accordingly, in an embodiment, the polypeptide of the present invention has an *in vivo* half-life that is greater than the equivalent polypeptide that does not comprise T307I, Q311R, M428L, N434L and/or Y436K mutations. In some such embodiments, the equivalent polypeptide comprises the H435R mutation, and optionally further comprises the T307R and A378V mutations. In an embodiment, the polypeptide of the present invention has an *in vivo* half-life that is greater than the equivalent polypeptide comprising an Fc region that does not comprise "I" at position 307, "R" at position 311, "L" at position 428, "L" at position 434 and/or "K" at position 436. In some such embodiments, the equivalent polypeptide comprises "R" at position 435, and optionally further comprises "R" at position 307 and "V" at position 378.

### Polypeptide forms

In an embodiment, the polypeptide of the invention further comprises a binding moiety that specifically binds to *Staphylococcus aureus.*

In an embodiment, the Fc region of the polypeptide of the invention is an immunoglobulin G (IgG) Fc region.

In an embodiment, the polypeptide of the invention is an antibody, an antigen-binding fragment thereof, or a polypeptide comprising a single domain antibody (VHH) fused to an Fc region. In an embodiment, the polypeptide comprises an Fc region and an antigen-binding domain, such as a domain comprising one or more variable immunoglobulin domains. In an embodiment, the polypeptide specifically binds to an *S*. *aureus* antigen.

In an embodiment, if the polypeptide of the present invention comprises one or more VH3 family antibody fragment (Fab) sequences, SpA does not bind to the VH3 Fab sequences. In an embodiment, SpA does not bind to the variable region of the polypeptide. In an embodiment, SpA does not bind to the Fab of the polypeptide. In an embodiment, SpA does not bind to the VHH domain of the polypeptide. In embodiments, this has no effect on the antibody-like specific binding of the variable region, which may specifically bind to *S*. *aureus.*

### Further forms of the invention

In an aspect, the present invention provides one or more nucleic acid sequences capable of expressing the polypeptide according to the invention.

In an embodiment, the nucleic acid is mRNA encoding the polypeptide according to the invention. In an embodiment, the nucleic acid is a vector encoding the polypeptide according to the invention.

In an embodiment, the nucleic acid encodes an antibody. In an embodiment, the nucleic acid encodes a polypeptide comprising a VHH domain and an Fc region.

In a further aspect, the invention provides a nucleic acid particle comprising the construct or nucleic acid sequences according to the invention. In one embodiment, the nucleic acid particle is a lipid nanoparticle (LNP).

In an aspect, the present invention provides a cell comprising the polypeptide or the one or more nucleic acid sequences according to the invention. In an embodiment, the cell is a mammalian cell. In an embodiment, the cell is a CHO cell. In an embodiment, the cell is a human cell.

Methods for engineering such cells are known in the art and include but are not limited to genetic modification of cells e.g. by transduction such as retroviral or lentiviral transduction, transfection (such as transient transfection - DNA or RNA based) including lipofection, polyethylene glycol, calcium phosphate and electroporation. Any suitable method may be used to introduce a nucleic acid sequence into a cell.

A cell of the present invention may be generated by introducing DNA or RNA coding for the polypeptide of the invention as defined herein by one of many means including transduction with a viral vector, transfection with DNA or RNA.

The cell of the invention may be made by introducing to a cell (e.g. by transduction or transfection) the one or more nucleic acids sequence according to the present invention.

The cell of the invention may comprise and/or secrete the polypeptide of the invention.

In an aspect, the present invention provides a composition comprising the polypeptide, the one or more nucleic acid sequences, the LNP or the cell according to the invention. The composition may be a pharmaceutical composition.

The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

### Methods of use and applications

In an aspect, the present invention provides an *in vitro* method comprising contacting a cell with the polypeptide, the one or more nucleic acid sequences, the LNP, the cell or the composition of the invention. In an embodiment, the cell is a mammalian cell. In an embodiment, the cell is a CHO cell. In an embodiment, the cell is a human cell.

In an aspect, the present invention provides the polypeptide, the one or more nucleic acid sequences, the LNP, the cell or the composition of the invention for use in a method of therapy or a diagnostic method.

In an aspect, the present invention provides the polypeptide, the one or more nucleic acid sequences, the LNP, the cell or the composition of the invention for use in the manufacture of a medicament for a therapy or a diagnostic agent.

In an aspect, the present invention provides a method of therapy or a diagnostic method comprising administering the polypeptide, the one or more nucleic acid sequences, the LNP, the cell or the composition of the invention to a subject.

In an embodiment, the therapy comprises treating or preventing a *Staphylococcus aureus* infection. In an embodiment, the therapy comprises treating or preventing a Streptococcal infection, particularly wherein the polypeptide has reduced affinity for SpG. In an embodiment, the *S*. *aureus* is resistant to one or more treatments. In some embodiments, the *S*. *aureus* is methicillin resistant. In an embodiment, the therapy is treating or preventing infection in a human. In an embodiment, treating or preventing comprises one or more of promoting *S. aureus* decolonization, preventing invasive disease caused by *S. aureus,* and improving the outcome of *S. aureus* bloodstream infections in the subject.

In an embodiment, the antibody is administered intravenously. In other embodiments, one or more nucleic acids encoding the antibody is/are administered. In an embodiment, the nucleic acid(s) may be introduced by transduction. In an embodiment, the nucleic acid(s) may be introduced by transfection.

### Method of manufacture

In an aspect, the present invention provides a method for making the polypeptide according to the invention, comprising expressing one or more nucleic acids capable of expressing the polypeptide. In an embodiment, the method comprises i) providing one or more nucleic acids capable of expressing the polypeptide and ii) expressing the nucleic acid(s) capable of expressing the polypeptide.

### Other general definitions

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino (N) to carboxy (C) orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

The term "polypeptide" is used in the conventional sense to mean a series of amino acids, typically L-amino acids, connected one to the other, typically by peptide bonds between the α-amino and carboxyl groups of adjacent amino acids. The term "polypeptide" is used interchangeably with the terms "amino acid sequence", "peptide" and/or "protein". The term "residues" is used to refer to amino acids in an amino acid sequence.

The term "variant" in relation to a polypeptide refers to a polypeptide that has an equivalent function to the amino acid sequences described herein, but which includes one or more amino acid substitutions, insertions or deletions.

The term "antibody" refers to an intact immunoglobulin of any isotype, or a fragment thereof that can compete with the intact antibody for specific binding to the target antigen, and includes chimeric, humanized, fully human, and bispecific antibodies. As used herein, the terms "antibody" or "immunoglobulin" are used interchangeably and refer to any of several classes of structurally related proteins that function as part of the immune response of an animal, including IgG, IgD, IgE, IgA, IgM, and related proteins, as well as polypeptides comprising antibody CDR domains that retain antigen-binding activity. "Fc region", "Fc domain" and "fragment crystallisable" region/domain are used interchangeably herein. The Fc region is a well-known part of an antibody, although the same Fc region may be present in other, antibody-like or antibody-derived polypeptides such as VHH-Fc fusion polypeptides. Herein, antibody residue numbering is according to the EU residue numbering scheme.

An amino acid residue in an antibody "corresponds" to a given residue when it occupies the same essential structural position within the antibody as the given residue. For example, a selected residue in a comparison antibody corresponds to position 428 (according to the EU numbering system as described herein) in an antibody provided herein when the selected residue occupies the same essential spatial or structural relationship to EU position 428 as assessed using applicable methods in the art. For example, a comparison antibody may be aligned for maximum sequence homology with the antibody provided herein and the position in the aligned comparison antibody that aligns with EU position 428 may be determined to correspond to it. Alternatively, instead of (or in addition to) a primary sequence alignment as described above, a three dimensional structural alignment can also be used, e.g., where the structure of the comparison antibody is aligned for maximum correspondence with an antibody provided herein and the overall structures compared. In this case, an amino acid that occupies the same essential position as EU position 428 in the structural model may be said to correspond.

The term "antigen" refers to a molecule or a portion of a molecule capable of being bound by a selective binding agent, such as an antibody. An antigen may possess one or more epitopes that are capable of interacting with different antibodies.

The term "epitope" includes any region or portion of molecule capable eliciting an immune response by binding to an immunoglobulin or to a T-cell receptor. Epitope determinants may include chemically active surface groups such as amino acids, sugar side chains, phosphoryl or sulfonyl groups, and may have specific three-dimensional structural characteristics and/or specific charge characteristics. Generally, antibodies specific for a particular target antigen will preferentially recognize an epitope on the target antigen within a complex mixture.

As used herein, the terms "polynucleotide", "nucleotide", "nucleic acid sequence" and "nucleic acid" are intended to be synonymous with each other.

The terms "variant", "homologue" or "derivative" in relation to a nucleotide sequence include any substitution of, variation of, modification of, replacement of, deletion of or addition of one (or more) nucleic acid from or to the sequence.

"Sequence identity" between two nucleic acid sequences indicates the percentage of nucleotides that are identical between the sequences. The terms "% identical" and "% identity" or similar terms are intended to refer, in particular, to the percentage of nucleotides or amino acids which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2, 482, with the aid of the local homology algorithm by Neddleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (e.g., at blast.ncbi.nlm.nih.gov/Blast.cgi?PAGE_TYPE=BlastSearch&BLAST_SPEC=blast2seq&LINK _LOC=align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment.

Percentage identity is obtained by determining the number of identical positions at which the sequences to be compared correspond, dividing this number by the number of positions compared (e.g., the number of positions in the reference sequence) and multiplying this result by 100.

In some embodiments, the degree of similarity or identity is given for a region which is at least about 50%, at least about 60%, at least about 70%, at least about 80%, at least about 90% or about 100% of the entire length of the reference sequence. For example, if the reference nucleic acid sequence consists of 200 nucleotides, the degree of identity is given for at least about 100, at least about 120, at least about 140, at least about 160, at least about 180, or about 200 nucleotides, in some embodiments continuous nucleotides. In some embodiments, the degree of similarity or identity is given for the entire length of the reference sequence.

In some embodiments, "isolated" means removed (e.g., purified) from the natural state or from an artificial composition, such as a composition from a production process. For example, a nucleic acid, peptide or polypeptide naturally present in a living animal is not "isolated", but the same nucleic acid, peptide or polypeptide partially or completely separated from the coexisting materials of its natural state is "isolated". An isolated nucleic acid, peptide or polypeptide can exist in substantially purified form, or can exist in a non-native environment such as, for example, a host cell.

The term "expression" as used herein is defined as the transcription and/or translation of a particular nucleotide sequence depending on the context. Typically, translation is required.

In the context of the present disclosure, the term "transcription" relates to a process, wherein the genetic code in a DNA sequence is transcribed into RNA (especially mRNA). Subsequently, the RNA may be translated into peptide or polypeptide.

With respect to RNA, the term "expression" or "translation" relates to the process in the ribosomes of a cell by which a strand of mRNA directs the assembly of a sequence of amino acids to make a peptide or polypeptide.

The term "nucleic acid" comprises deoxyribonucleic acid (DNA), ribonucleic acid (RNA), combinations thereof, and modified forms thereof. The term comprises genomic DNA, cDNA, mRNA, recombinantly produced and chemically synthesized molecules. A nucleic acid may be present as a single-stranded or double-stranded and linear or covalently circularly closed molecule. A nucleic acid can be isolated. The term "isolated nucleic acid" means, according to the present disclosure, that the nucleic acid (i) was amplified *in vitro,* for example via polymerase chain reaction (PCR) for DNA or in vitro transcription (using, e.g., an RNA polymerase) for RNA, (ii) was produced recombinantly by cloning, (iii) was purified, for example, by cleavage and separation by gel electrophoresis, or (iv) was synthesized, for example, by chemical synthesis.

The term "nucleoside" (abbreviated herein as "N") relates to compounds which can be thought of as nucleotides without a phosphate group. While a nucleoside is a nucleobase linked to a sugar (e.g., ribose or deoxyribose), a nucleotide is composed of a nucleoside and one or more phosphate groups. Examples of nucleosides include cytidine, uridine, pseudouridine, adenosine, and guanosine.

The five standard nucleosides which usually make up naturally occurring nucleic acids are uridine, adenosine, thymidine, cytidine and guanosine. The five nucleosides are commonly abbreviated to their one letter codes U, A, T, C and G, respectively. However, thymidine is more commonly written as "dT" ("d" represents "deoxy") as it contains a 2'-deoxyribofuranose moiety rather than the ribofuranose ring found in uridine. This is because thymidine is found in deoxyribonucleic acid (DNA) and not ribonucleic acid (RNA). Conversely, uridine is found in RNA and not DNA. The remaining three nucleosides may be found in both RNA and DNA. In RNA, they would be represented as A, C and G, whereas in DNA they would be represented as dA, dC and dG.

A modified purine (A or G) or pyrimidine (C, T, or U) base moiety is preferably modified by one or more alkyl groups, more preferably one or more C1-4 alkyl groups, even more preferably one or more methyl groups. Particular examples of modified purine or pyrimidine base moieties include N7-alkyl-guanine, N6-alkyl-adenine, 5-alkyl-cytosine, 5-alkyl-uracil, and N(1)-alkyl-uracil, such as N7-C1-4 alkyl-guanine, N6-C1-4 alkyl-adenine, 5-C1-4 alkyl-cytosine, 5-C1-4 alkyl-uracil, and N(1)-C1-4 alkyl-uracil, preferably N7-methyl-guanine, N6-methyl-adenine, 5-methyl-cytosine, 5-methyl-uracil, N1-methyl-pseudouridine, and N(1)-methyl-uracil.

Herein, the term "DNA" relates to a nucleic acid molecule which includes deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

DNA may be recombinant DNA and may be obtained by cloning of a nucleic acid, in particular cDNA. The cDNA may be obtained by reverse transcription of RNA.

The term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered/modified nucleotides can be referred to as analogs of naturally occurring nucleotides, and the corresponding RNAs containing such altered/modified nucleotides (i.e., altered/modified RNAs) can be referred to as analogs of naturally occurring RNAs. A molecule contains "a majority of ribonucleotide residues" if the content of ribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

"RNA" includes mRNA, tRNA, ribosomal RNA (rRNA), small nuclear RNA (snRNA), self-amplifying RNA (saRNA), single-stranded RNA (ssRNA), dsRNA, inhibitory RNA (such as antisense ssRNA, small interfering RNA (siRNA), or microRNA (miRNA)), activating RNA (such as small activating RNA) and immunostimulatory RNA (isRNA). In some embodiments, "RNA" refers to mRNA.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of" also include the term "consisting of'.

The terms "equivalent to", "similar to" or "comparable to" as used herein are interchangeable with "not substantially different from" and, in a stricter embodiment, "not different from" or "the same as". It will be understood herein that the margin allowable within e.g. "substantially" is that which would be understood by the skilled person as not having a significant effect on the function in question. E.g., the polypeptide of the present invention which has an *in vivo* half-life that is equivalent to that of a wild-type polypeptide can be considered to have an *in vivo* half-life that is not substantially different from that of the equivalent wild-type polypeptide. In this embodiment, any minor difference between the *in vivo* half-life of the mutant and the wild-type polypeptide is not significant and does not alone make any substantial difference to the *in vivo* function of the polypeptides.

References herein to any function or effect in an immune system, *in vivo,* in a cell or in an organism are preferably in a human or human cell, as applicable.

The publications discussed herein are provided solely for their disclosure prior to the filing date of the present application. Nothing herein is to be construed as an admission that such publications constitute prior art to the claims appended hereto.

The invention will now be further described by way of Examples, which are meant to serve to assist one of ordinary skill in the art in carrying out the invention and are not intended in any way to limit the scope of the invention.

### Numbered embodiments of the invention

1. A polypeptide comprising a fragment crystallisable (Fc) region having reduced affinity for *Staphylococcus aureus* protein A (SpA), wherein the polypeptide sequence of the Fc region comprises one or more of the following substitution mutations: T307I, Q311R, M428L, N434L and Y436K.
2. The polypeptide of embodiment 1, wherein the polypeptide sequence of the Fc region comprises the substitution mutation M428L, and optionally comprises one or more of T307I, Q311R, N434L and Y436K.
3. The polypeptide of embodiment 1 or 2, wherein the polypeptide sequence of the Fc region comprises all of the substitution mutations T307I, Q311R, M428L, N434L and Y436K.
4. The polypeptide of any one of embodiment s 1 to 3, wherein the polypeptide does not comprise the substitution mutation H435R.
5. The polypeptide of any one of embodiments 1 to 4, wherein the amino acid position numbering corresponds to EU numbering.
6. The polypeptide of any one of embodiments 1 to 5, wherein the Fc region of the polypeptide has reduced affinity for the second immunoglobulin-binding protein of *Staphylococcus aureus* (Sbi).
7. The polypeptide of any one of embodiments 1 to 6, wherein the Fc region of the polypeptide has reduced affinity for Streptococcal protein G (SpG).
8. The polypeptide of any one of embodiments 1 to 7, wherein the Fc region of the polypeptide has reduced affinity for SpA, Sbi and/or SpG relative to the Fc region of the polypeptide that does not comprise the substitution mutations.
9. The polypeptide of any one of embodiments 1 to 8, wherein the Fc-mediated effector functions of the polypeptide are not inhibited in the presence of *Staphylococcus aureus.*
10. The polypeptide of any one of embodiments 1 to 9, wherein the Fc region of the polypeptide binds to the neonatal Fc receptor (FcRn).
11. The polypeptide of any one of embodiments 1 to 10, wherein the polypeptide has an *in vivo* half-life that is equivalent to the *in vivo* half-life of the polypeptide comprising an Fc region that does not comprise the substitution mutations.
12. The polypeptide of any one of embodiments 1 to 11, wherein the Fc region of the polypeptide binds to the Fc-gamma receptor (FcγR).
13. The polypeptide of any one of embodiments 1 to 12, wherein the polypeptide has improved complement component 1q (C1q) recruitment.
14. The polypeptide of any one of embodiments 1 to 13, wherein the polypeptide further comprises a binding moiety that specifically binds to *Staphylococcus aureus.*
15. The polypeptide of any one of embodiments 1 to 14, wherein the Fc region of the polypeptide is an immunoglobulin G (IgG) Fc region.
16. The polypeptide of any one of embodiments 1 to 15, wherein the polypeptide is an antibody, an antigen-binding fragment thereof, or a polypeptide comprising a single domain antibody (VHH) fused to an Fc region.
17. One or more nucleic acid sequences capable of expressing the polypeptide according to any one of embodiments 1 to 16.
18. The one or more nucleic acid sequences of embodiment 17, wherein the nucleic acid is mRNA encoding the polypeptide according to any one of embodiments 1 to 16.
19. A cell comprising the polypeptide or the one or more nucleic acid sequences according to any one of embodiments 1 to 18.
20. A composition comprising the polypeptide, the one or more nucleic acid sequences or the cell according to any one of embodiments 1 to 19.
21. An *in vitro* method comprising contacting a cell with the polypeptide, the one or more nucleic acid sequences, the cell or the composition of any one of embodiments 1 to 20.
22. The polypeptide, the one or more nucleic acid sequences, the cell or the composition of any one of embodiments 1 to 20 for use in a method of therapy or a diagnostic method.
23. The polypeptide, the one or more nucleic acid sequences, the cell or the composition of any one of embodiments 1 to 20 for use in the manufacture of a medicament for a therapy or a diagnostic agent.
24. A method of therapy or a diagnostic method comprising administering the polypeptide, the one or more nucleic acid sequences, the cell or the composition of any one of embodiments 1 to 20 to a subject.
25. The method of any one of embodiments 22 to 24, wherein the therapy is treating or preventing a *Staphylococcus aureus* infection.
26. A method for making the polypeptide according to any one of embodiments 1 to 16, comprising expressing one or more nucleic acids capable of expressing the polypeptide.

### Examples

### Example 1 - Screening for Fc variants

For an effective use of a therapeutic antibody in *S. aureus* infections, the Fc-mediated capture of the antibody must be inhibited. To achieve this, the present inventors identified novel mutations in the Fc-domain to inhibit SpA-mediated binding.

### In silico screening for FcRn affinity, SpA affinity and stability

A considerable challenge presented in the screening process was the highly overlapping binding sites of SpA/Sbi and the neonatal Fc receptor (FcRn). FcRn mediates antibody recycling via pH-dependent rescue from endosomal degradation, and is thus essential for the long serum half-life of antibodies. Both SpA/Sbi and FcRn bind to IgG-Fc-domains at the interface of CH2 and CH3 regions and partly involve the same amino acids. Using advanced *in silico* Al-driven screening techniques, the protein complexes Fc:FcRn and Fc:SpA were modelled *in silico* for a large number of Fc variants, and mutagenesis and stability predictions led to the identification of 180 candidate Fc mutants.

### Expression of screening candidates as VHH-Fc-fusions

The 180 identified candidate Fc mutants, along with controls from literature (Chen et al., PNAS 2022) were subsequently expressed and characterized in the format of VHH-Fc-fusions with a VHH targeting a placeholder target antigen (i.e. for later replacement with S. aureus-specific VHHs). The characterisation included testing for both SpA- and FcRn-binding. Of the 180 candidate proteins and the controls from the literature, 32 proteins were further characterized with respect to their functional capacities. The other candidates did not pass initial testing of SpA- and/or FcRn-binding. Through several rounds of testing, 3 candidate mutants of further interest were identified: Fc049, Fc067 and Fc136, as well as two control mutants from literature: Fc183 and Fc185. Details of the Fc domains of these proteins are provided in Table 1.

**TABLE 1: Candidate mutants and literature controls after initial screening**

| **ID** | **Mutations** | **Fc Domain Amino Acid Sequence** | **Source** |
|---|---|---|---|
| **Fc049** | M252S | SVFLFPPKPKDTLSISRNPEVTCVVVDVSHEDPEVKFNWYV DGVEVHNAKTKPREEQYNSTYRVVSVLTVLHRDWLNGKEY KCKVSNKALPAPIEKTISKAKGQPREPQVYTLPPSRDELTKN QVSLTCLVKGFYPSDIAVEWKSNGQPENNYKTTPPVLDSDG SFFLYSKLTVDKSRWQQGNVFSCSVMHEALHNHKTQKSL (SEQ ID NO: 1) | Screening |
| | T256N | | |
| | Q311R | | |
| | E382 K | | |
| | Y436K | | |
| **Fc067** | Q311R | SVFLFPPKPKDTLMISRTPEVTCVVVDVSHEDPEVKFNWYV DGVEVHNAKTKPREEQYNSTYRVVSVLTVLHRDWLNGSEY KCKVSNKALPAPIEKTISKAKGQPREPQVYTLPPSRDELTKN QVSLTCLVKGFYPSDIAVEWESNGAPENNYKTTPPVLDSDG SFFLYSKLTVDKSRWQQGNVFSCSVMHEALHLHETQKSL | Screening |
| | K317S | | |
| | Q386A | | |
| | N434L | | |
| | Y436E | | |
| | | (SEQ ID NO: 2) | |
| **Fc136** | T307I | SVFLFPPKPKDTLMISRTPEVTCVVVDVSHEDPEVKFNWYV DGVEVHNAKTKPREEQYNSTYRVVSVLIVLHRDWLNGKEY KCKVSNKALPAPIEKTISKAKGQPREPQVYTLPPSRDELTKN QVSLTCLVKGFYPSDIAVEWESNGQPENNYKTTPPVLDSDG SFFLYSKLTVDKSRWQQGNVFSCSVLHEALHLHKTQKSL (SEQ ID NO: 3) | Screening |
| | Q311R | | |
| | M428L | | |
| | N434L | | |
| | Y436K | | |
| **Fc183** | H435R | SVFLFPPKPKDTLMISRTPEVTCVVVDVSHEDPEVKFNWYV DGVEVHNAKTKPREEQYNSTYRVVSVLTVLHQDWLNGKEY KCKVSNKALPAPIEKTISKAKGQPREPQVYTLPPSREEMTKN QVSLTCLVKGFYPSDIAVEWESNGQPENNYKTTPPVLDSDG SFFLYSKLTVDKSRWQQGNVFSCSVMHEALHNRYTQKSL (SEQ ID NO: 4) | Chen et al., PNAS 2022 |
| **Fc185** | H435R | SVFLFPPKPKDTLMISRTPEVTCVVVDVSHEDPEVKFNWYV DGVEVHNAKTKPREEQYNSTYRVVSVLRVLHQDWLNGKEY KCKVSNKALPAPIEKTISKAKGQPREPQVYTLPPSREEMTKN QVSLTCLVKGFYPSDIVVEWESNGQPENNYKTTPPVLDSDG SFFLYSKLTVDKSRWQQGNVFSCSVMHEALHNRYTQKSL (SEQ ID NO: 5) | Chen et al., PNAS 2022 |
| | T307R | | |
| | A378V | | |

Notably, the candidate mutants identified in Table 1 did not comprise any of the same substitution mutations as the control mutants from the literature (although some of the amino acid positions are the same, the specific substitution mutations are not the same). Notably, none of the candidate mutants identified in Table 1 possessed the H435R mutation of the control mutants. Notably, Fc136 was the sole mutant identified (out of any of the 180 screened mutants) to possess the M428L mutation.

### Expression and stability tests

The expression and stability of the screening candidate mutants and control mutants were tested, and results were analysed from a western blot.

All the tested screening candidate mutants could be well expressed. The selected lead screening candidate mutants listed in Table 1 showed no aberrant fragmentation in the western blot. However, Fc183 showed a smear in the western blot, indicating possible liabilities with the expression and stability of this protein.

### FcRn binding tests

FcRn binding of the Fc mutants listed in Table 1 was tested via ELISA at pH 6 and at pH 7. Some additional variants from the literature were also tested. For efficient antibody recycling within the cell, it is advantageous for FcRn binding to occur at pH 6, but for binding to be weaker at pH 7.

At pH 6, the Fc mutants listed in Table 1 displayed binding to FcRn which was similar to the WT control (the unmodified VHH-Fc fusion). Binding to FcRn at pH 6 was considered to be acceptable for all tested mutants of Table 1.

However, two additional Fc mutants from the literature were tested and failed in this screening stage, as they exhibited weak binding to FcRn at pH 6. Fc182 ("AESP" from Chen et al. (2022), comprising the mutations S254A, Q311E, L432S and N434P) and Fc184 ("AESP-RV" from Chen et al. (2022), comprising the mutations S254A, Q311E, L432S, N434P, T307R and A378V) both exhibited unacceptably weak binding to FcRn at pH 6.

At pH 7, binding to FcRn was considered to be acceptable for all of the Fc mutants listed in Table 1, with the notable exception of Fc185. Fc185 showed strong binding to FcRn at pH 7, to a degree which may negatively impact the antibody half-life of Fc185. This result is shown in Figure 1.

Further, two additional Fc mutants from the literature were tested and failed in this screening stage, as they exhibited strong binding to FcRn at pH 7. Fc186 ("R-QVV" from Chen et al. (2022), comprising the mutations H435R, T307Q, Q311V and A378V) and Fc187 ("R-DDRVV" from Chen et al. (2022), comprising the mutations H435R, T256D, N286D, T307R, Q311V and A378V) both exhibited unacceptably strong binding to FcRn at pH 7, indicative of a shorter serum half-life.

### Example 2 - Testing antibody effector functions

### FcγR receptor binding tests

Antibody effector functions are mostly mediated by the interaction of the Fc-domain with relevant Fc-receptors. In the case of IgG antibodies, the Fc-domain of which was used in these experiments, Fcγ-Receptors (FcγRs) are particularly relevant. As an approximation of Fc-effector functionality, binding to Fcγ-Receptors was tested via ELISA (see Figure 2).

Binding to Fcγ-Receptors for the screening candidate mutant Fc136 was comparable to the wildtype control, indicating that the inserted mutations did not negatively affect Fcγ-Receptor binding. Binding was conserved for activating receptors, such as FcγRl (Figure 2A), FcγRlla (Figure 2B) and FcγRIIIa (Figure 2D), as well as inhibitory receptors, such as FcγRIIb/c (Figure 2C). High affinity polymorphisms of FcγRlla and FcγRllla were used in these tests.

The literature mutant Fc185 exhibited the lowest maximum effect for both FcγRl and FcγRlla. The literature mutant Fc183 exhibited the lowest maximum effect for FcγRllb/c.

These tests confirmed that the mutations in Fc136 did not significantly reduce FcγR binding.

### Functional testing of Fc mutants

The Fc mutants listed in Table 1 were subjected to functional testing, including cell binding testing, an ADCC assay, an ADCP assay and a C1q recruitment assay.

Cell binding was assessed with a target antigen over-expressing Flpln CHO cell line, an experimentally validated cell line based on the commercially available Flpln CHO system (https://www.thermofisher.com/order/catalog/product/de/en/R75807). The ADCC assay was carried out with a kit obtained from Promega GmbH (https://www.promega.de/en/products/reporter-bioassays/fc-effector-activity-bioassays/adcc-bioassays/?catNum=G7010). The ADCP assay was also carried out with a kit obtained from Promega GmbH (https://www.promega.de/en/products/reporter-bioassays/fc-effector-activity-bioassays/adcp-bioassays/?catNum=G9991). The C1q recruitment assay was designed based on available literature (Pawluczkowvcz et al., J Immunol 1;183(1):749-58 (2009) [Pubmed ID: 19535640]).

The results of the functional tests are shown in Figure 3.

It was determined that cell surface binding to the target antigen over-expressing cell line was unaffected by the inserted mutations (Figure 3C).

However, some differences in Fc-effector functions were observed. Of note, Fc136 performed at least comparably to the wildtype protein in ADCC and ADCP reporter assays. Moreover, Fc136 showed favourably strong signals in the C1q recruitment assay. It was also noted that Fc049 showed ADCC signals but the signals were weaker than those of Fc136.

These tests confirmed that the mutations did not significantly reduce cell binding. The tests further confirmed that Fc136 performed well in all assays, and that Fc136 showed favourably strong signals in the C1q recruitment assay, compared to the other tested mutants. C1q recruitment to target cells bound by IgG antibodies/VHH-Fc fusion proteins etc. initiates the classical pathway of complement, thereby promoting complement-dependent cytotoxicity (CDC). Thus, these tests indicate that Fc136 may induce enhanced CDC.

### Example 3 - Testing for inhibited Sbi, SpA and SpG binding

The Fc mutants including Fc136 were further tested via ELISA for inhibition of binding to the second immunoglobulin-binding protein of *Staphylococcus aureus* (Sbi), as well as SpA and SpG, in comparison with a wild-type control (Fc001/WT).

The ELISA results are shown in Figure 4. Fc136 showed inhibited Sbi, SpA and SpG binding, indicating that Fc136 has reduced affinity for Sbi, SpA and SpG. In particular, Fc136 has substantially reduced affinity for Sbi, SpA and SpG relative to the wild-type equivalent protein.

### Example 4 - Further confirmation with purified polypeptides

The VHH-Fc fusion mutants Fc136, Fc183 and Fc185 and a wild-type control (WT) were further tested in purified forms. This was done to further confirm that the Fc136 mutant is no longer bound to by Staphylococcal Protein A and Streptococcal Protein G, whilst maintaining other properties that are similar to that of the wild-type control (and are therefore desirable).

### Protein production

VHH-Fc-fusions were produced via transient transfection of ExpiCHO cells. The cell culture supernatant was harvested 8 days post-transfection and subsequently purified via affinity chromatography (FLAG-tag) and preparative size exclusion chromatography. This yielded protein solutions of high purity (> 95%, confirmed via SDS-PAGE), with the target protein running at a molecular weight of around 95 kDa under non-reducing conditions and around 45 kDa under reducing conditions. Reducing conditions led to reduction of the disulfide bonds in the hinge-region of the antibody constructs and thus to single protein chains. Proteins were normalized to 2 mg/mL in sterile PBS.

### ELISA testing interactions with target antigen, Fc-receptors, SpA, SpG and Sbi

Uniform binding of the VHH-Fc-fusions was detected on the target antigen, with EC50 values ranging from 0.39 to 0.51 nM (Figure 5A).

Binding to FcγRl (CD64), FcγRlla (CD32a) and FcγRllla (CD16) was comparable between Fc136 and the WT (Fc001) (Figures 5B-D).

All candidate Fc-mutants (Fc136, Fc183, Fc185) displayed reduced binding to Protein A, with binding only being detected in very high concentrations - EC50 values were increased by > factor 1000 (Figure 6A).

In contrast, only Fc136 showed reduced binding to Streptococcal Protein G, whereas the H435R-comprising candidates - Fc183 and Fc185 - showed comparable binding to Protein G as the WT (Figure 6B).

Further, all candidate Fc-mutants (Fc136, Fc183, Fc185) displayed reduced binding to Sbi, again with binding only being detected in very high concentrations (Figure 6A).

In short, further studies with purified polypeptides confirmed that the Fc136 mutant VHH-Fc fusion displayed reduced affinity for SpA, SpG and Sbi, whilst maintaining the same desired properties of target antigen and Fc-receptor binding as that of the wild-type VHH-Fc fusion. In particular, Fc136 has substantially reduced affinity for SpA, SpG and Sbi relative to the equivalent wildtype polypeptide.

### Example 5 - Improved half-life relative to wild-type and literature polypeptides

To assess binding of the identified Fc-mutants to human FcRn *in vivo,* a pharmacokinetics experiment was conducted. For this, NOD.Cg-*Fcgrt^{tm1Dcr} Prkdc^{scid} Il2rg^{tm1Wjl}* Tg(FCGRT)32Dcr/J (NSG FcRn-/- hFcRn (32) Tg) mice expressing human instead of murine FcRn were selected as a model organism, since binding of the Fc mutants to human versus murine FcRn showed different patterns.

5 groups, each including 4 mice, were injected with one of the following samples:
- Fc136: 250 µg in 150 µL PBS
- Fc183: 250 µg in 150 µL PBS
- Fc185: 250 µg in 150 µL PBS
- Fc001: 250 µg in 150 µL PBS
- PBS: 150 µL

Blood was drawn at different time points across a timeframe of 18 days. VHH-Fc concentrations in serum were subsequently quantified via ELISA, leading to the results shown in Figure 7 and Table 1 below.

Fc136 consistently showed the highest serum concentrations in comparison to all other samples tested. Serum concentrations of Fc136 were significantly higher than those observed for the respective WT protein (Fc001). Fc136 also showed significantly higher concentrations than Fc185. Fc183 did not differ substantially in half-life and serum concentrations from the wildtype.

**Table 2: Serum half-lives of Fc mutants as determined via Two-Phase Decay Model**

| ***Protein*** | ***Serum half-life [h]*** |
|---|---|
| Fc001 | 70.50 |
| Fc136 | 92.03 |
| Fc183 | 72.25 |
| Fc185 | 81.51 |

## Claims

1. A polypeptide comprising a fragment crystallisable (Fc) region having reduced affinity for *Staphylococcus aureus* protein A (SpA), wherein the polypeptide sequence of the Fc region comprises one or more of the following substitution mutations: T307I, Q311R, M428L, N434L and Y436K.

2. The polypeptide of claim 1, wherein the polypeptide sequence of the Fc region comprises the substitution mutation M428L, and optionally comprises one or more of T307I, Q311R, N434L and Y436K.

3. The polypeptide of claim 1 or 2, wherein the polypeptide sequence of the Fc region comprises all of the substitution mutations T307I, Q311R, M428L, N434L and Y436K.

4. The polypeptide of any one of claims 1 to 3, wherein the polypeptide does not comprise the substitution mutation H435R.

5. The polypeptide of any one of claims 1 to 4, wherein the Fc region of the polypeptide has reduced affinity for the second immunoglobulin-binding protein of *Staphylococcus aureus* (Sbi), optionally wherein the Fc region of the polypeptide has reduced affinity for Streptococcal protein G (SpG).

6. The polypeptide of any one of claims 1 to 5, wherein the Fc region of the polypeptide has reduced affinity for SpA, Sbi and/or SpG relative to the Fc region of the polypeptide that does not comprise the substitution mutations, optionally wherein the Fc-mediated effector functions of the polypeptide are not inhibited in the presence of *Staphylococcus aureus.*

7. The polypeptide of any one of claims 1 to 6, wherein the Fc region of the polypeptide binds to the neonatal Fc receptor (FcRn), optionally wherein the polypeptide has an *in vivo* half-life that is equivalent to the *in vivo* half-life of the polypeptide comprising an Fc region that does not comprise the substitution mutations.

8. The polypeptide of any one of claims 1 to 7, wherein the Fc region of the polypeptide binds to the Fc-gamma receptor (FcγR), optionally wherein the polypeptide has improved complement component 1q (C1q) recruitment.

9. The polypeptide of any one of claims 1 to 8, wherein the polypeptide further comprises a binding moiety that specifically binds to *Staphylococcus aureus,* optionally wherein the Fc region of the polypeptide is an immunoglobulin G (IgG) Fc region.

10. The polypeptide of any one of claims 1 to 9, wherein the polypeptide is an antibody, an antigen-binding fragment thereof, or a polypeptide comprising a single domain antibody (VHH) fused to an Fc region.

11. One or more nucleic acid sequences capable of expressing the polypeptide according to any one of claims 1 to 10, wherein the nucleic acid is optionally mRNA encoding the polypeptide according to any one of claims 1 to 10.

12. A cell comprising the polypeptide or the one or more nucleic acid sequences according to any one of claims 1 to 11.

13. A composition comprising the polypeptide, the one or more nucleic acid sequences or the cell according to any one of claims 1 to 12.

14. An *in vitro* method comprising contacting a cell with the polypeptide, the one or more nucleic acid sequences, the cell or the composition of any one of claims 1 to 13.

15. The polypeptide, the one or more nucleic acid sequences, the cell or the composition of any one of claims 1 to 14 for use in a method of therapy or a diagnostic method, optionally wherein the therapy is treating or preventing a *Staphylococcus aureus* infection.
